# EUROPÄISCHE PATENTANMELDUNG

(11) **EP 1 702 629 A1**
(43) Veröffentlichungstag der Anmeldung: **20.09.2006**
(21) Anmeldenummer: 06004570.5
(22) Anmeldetag: 07.03.2006
(51) Int. Cl.: A61L 2/02, A61L 2/03, B60H 3/00

(54) **Vorrichtung zur Abgabe von Duftstoffen**

(30) Priorität: 09.03.2005 DE 102005010713
(71) Anmelder: Tradesetter AG, 8853 Lachen (CH)
(72) Erfinder: Reichmuth, Erich, 8853 Lachen (CH)
(74) Vertreter: Haft, Uwe Michael

(57) **Zusammenfassung**

Eine Vorrichtung zur Abgabe von Duftstoffen besteht aus einem Gehäuse (28) mit einem Vorratsbehälter (2) für die Duftstofflösung (5), einer mit einer elektrischen Heizeinrichtung (24) erwärmbaren Verdampfungsschale (8), einer elektrochemischen Gasentwicklungszelle (7), durch die die Duftstofflösung (5) durch Verdrängung aus dem Vorratsbehälter (2) der Verdampfungsschale (8) zugeführt wird, und einem Ventilator (37) über der Verdampfungsschale (8). Der Vorratsbehälter (2) mit der Duftstofflösung (5), die elektrochemische Gasentwicklungszelle (7) und die Verdampfungsschale (8) sind zusammen als austauschbare Kartusche (1) ausgebildet und das Gehäuse (28) nimmt wenigstens eine solche austauschbare Kartusche (1) auf.

## Beschreibung

Die Erfindung bezieht sich auf eine Vorrichtung zur Abgabe von Duftstoffen nach dem Oberbegriff des Anspruchs 1.

Eine solche Vorrichtung ist aus der nicht veröffentlichten deutschen Patentanmeldung 103 53 286.2 bekannt. Dabei ist bereits eine austauschbare Einheit vorgesehen, die aus dem aus einem Zylinder bestehenden Vorratsbehälter für die Duftstofflösung, der Gasentwicklungszelle und einem im Kolben geführten Zylinder besteht, der von dem Gas der Gasentwicklungszelle beaufschlagt die Duftstofflösung in die Verdampfungsschale drückt.

In vielen Einrichtungen, beispielsweise einem Hotel, wird der Duft einer solchen Beduftungsvorrichtung häufig gewechselt; z.B. wird zum Frühstück ein anderer Duft als zu den anderen Mahlzeiten oder anderen Anlässen verwendet. Die Vorrichtung nach der älteren Patentanmeldung 103 53 286.2 hat den Nachteil, dass bei jedem Duftwechsel die Verdampfungsschale sorgfältig gereinigt werden muss.

Aufgabe der Erfindung ist es, den Betrieb einer solchen Beduftungsvorrichtung wesentlich zu vereinfachen.

Dies wird erfindungsgemäß durch die im Anspruch 1 gekennzeichnete Vorrichtung erreicht. In den Unteransprüchen sind vorteilhafte Ausgestaltungen der Erfindung wiedergegeben.

Nach der Erfindung sind der Vorratsbehälter mit der Duftstofflösung, die elektrochemische Gasentwicklungszelle und die Verdampfungsschale zusammen als austauschbare Kartusche ausgebildet. Damit braucht beim Duftwechsel nur die Kartusche ausgewechselt zu werden.

Die Kartusche besteht aus dem vorzugsweise zylindrisch ausgebildeten Vorratsbehälter und einem damit lösbar oder unlösbar verbundenen Aufsatz, der die Gasentwicklungszelle enthält und an seiner Oberseite die Verdampfungsschale aufweist. Der Aufsatz kann dabei eine zylindrische Umfangswand aufweisen, die dem Durchmesser des Vorratsbehälters entspricht. Der Aufsatz kann aus Kunststoff bestehen und beispielsweise durch Spritzguss hergestellt sein. Der Vorratsbehälter besteht vorzugsweise aus Blech oder Kunststoff.

Der Vorratsbehälter weist an seiner Oberseite eine Öffnung auf. Diese Öffnung kann einen umgebördelten Rand aufweisen. In die Vorratsbehälteröffnung greift vorzugsweise ein Kragen an dem Boden des Aufsatzes gasdicht ein. Innerhalb des Kragens weist der Boden des Aufsatzes normalerweise zwei Öffnungen auf. Durch die eine Öffnung ist die Gasentwicklungszelle mit dem Inneren des Vorratsbehälters verbunden, so dass das Gas aus der Gasentwicklungszelle in den Vorratsbehälter strömen kann. Durch die andere Öffnung im Boden des Aufsatzes wird die Duftstofflösung von dem Vorratsbehälter zu dem Verdampfer geführt, beispielsweise über ein Steigrohr oder dergleichen Steigleitung.

Das Gehäuse der erfindungsgemäßen Vorrichtung, das die Kartusche aufnimmt, ist vorzugsweise als Säule ausgebildet. Die elektrochemische Gasentwicklungszelle wird vorzugsweise durch eine Stromversorgung betrieben, die im Gehäuse, vorzugsweise im Bereich des Bodens des Gehäuses angeordnet ist. Diese Energieversorgung kann eine Batterie, ein Akku oder dergleichen netzunabhängige Energiequelle oder ein Anschluss an das Stromnetz sein. Die elektrochemische Gasentwicklungszelle besteht aus einer Elektrode, einer Gegenelektrode und einem Elektrolyt. Wenn ein wässeriger Elektrolyt verwendet wird, wird Wasserstoff- oder Sauerstoffgas entwickelt. Eine derartige Gasentwicklungszelle geht z.B. aus US 5,242,565 hervor.

Der Aufsatz weist unterhalb der Verdampfungsschale einen Freiraum auf, in den die elektrische Heizeinrichtung zur Erwärmung der Verdampfungsschale eingeführt wird.

Die Heizeinrichtung kann dazu auf einer Zunge angeordnet sein, die durch einen Einsteckschlitz in der Umfangswand des Aufsatzes in den Freiraum eingeführt wird. Die elektrische Heizeinrichtung kann beispielsweise durch eine Platine auf der Zunge gebildet sein, die mit dem Boden der Verdampfungsschale in Berührung steht.

Der Teil des Aufsatzes, der die Verdampfungsschale bildet, kann ebenfalls aus Kunststoff gebildet sein, wobei vorzugsweise ein gut wärmeleitender Kunststoff verwendet wird.

Die elektrochemische Gasentwicklungsquelle wird vorzugsweise ebenfalls über diese Zunge an die Energieversorgung in dem Gehäuse der Vorrichtung angeschlossen. Die Zunge kann dazu mit einem entsprechenden elektrischen Kontakt versehen sein.

Mit der Steuerschaltung, die ebenfalls vorzugsweise im oder am Gehäuse vorgesehen ist, wird die elektrische Heizeinrichtung und die Stromzufuhr zu der elektrochemischen Gasentwicklungszelle gegebenenfalls über einen Timer gesteuert.

Nachstehend ist die Erfindung anhand der beigefügten Zeichnung beispielhaft näher erläutert. Darin zeigen jeweils schematisch:
- Figur 1: eine perspektivische Ansicht einer Kartusche;
- Figur 2: einen Längsschnitt durch die Kartusche nach Figur 1, mit teilweise weggebrochenem Vorratsbehälter, und durch die Zunge an der Gehäuseinnenwand; und
- Figur 3: eine Ansicht auf das Gehäuse mit abgenommenem Verschlussdeckel.

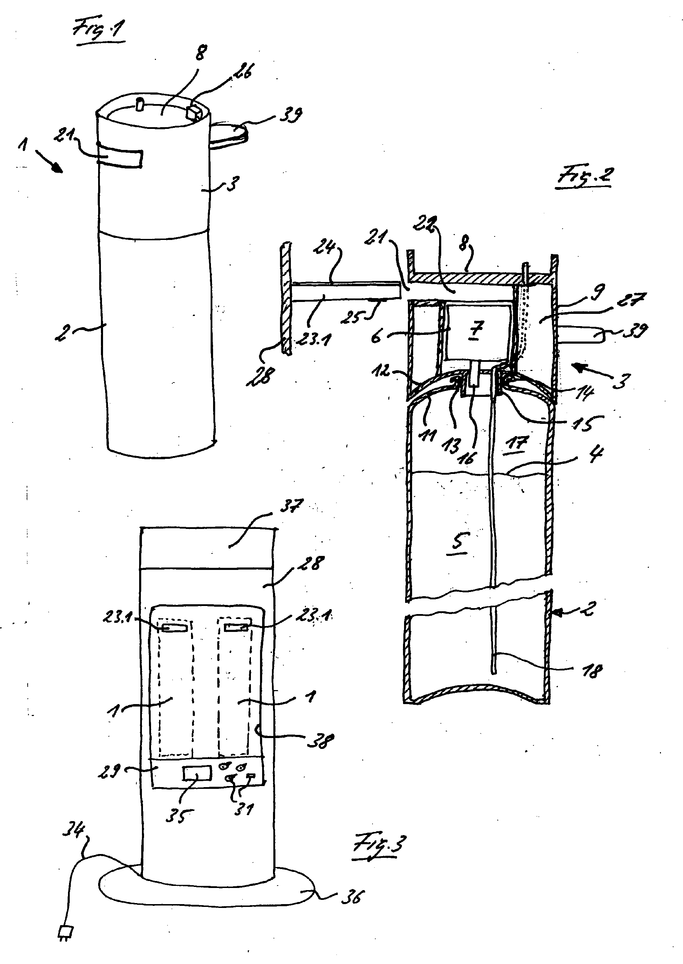

Gemäß Figur 1 und 2 besteht die Kartusche 1 aus einem dosenförmigen Vorratsbehälter 2 und einem zylinderförmigen Aufsatz 3. Der Behälter 2 ist bis zum Niveau 4 mit einer Duftstofflösung 5 gefüllt. Der Behälter 2 besteht z.B. aus Aluminiumblech.

In dem als Kappe ausgebildeten Aufsatz 3, der z.B. aus Kunststoff besteht, ist in einem Innengehäuse 6 eine elektrochemische Gasentwicklungszelle 7 angeordnet. Die Oberseite der Kappe 3 ist als Verdampfungsschale 8 ausgebildet. Der Durchmesser der zylindrischen Umfangswand 9 des Aufsatzes 3 entspricht dem Durchmesser des Behälters 2.

Der Behälter 2 ist an seiner Oberseite 11 konvex gewölbt ausgebildet. Der Boden 12 des Aufsatzes 3 ist mit einer entsprechenden Wölbung konkav ausgebildet.

In der Mitte seiner Oberseite 11 weist der Behälter 2 eine Öffnung 13 auf, deren Rand 14 nach außen umgebördelt ist.

In der Mitte seines Bodens 12 ist der Aufsatz 3 mit einem nach unten ragenden Kragen 15 versehen, der dicht an dem umgebördelten Rand 14 der Öffnung 13 anliegt.

Die elektrochemische Gasentwicklungszelle 7 weist innerhalb des Kragens 15 eine Austrittsöffnung 16 auf, so dass das Gas in dem Gasraum 17 oberhalb des Niveaus 4 in den Behälter 2 strömen kann.

Eine als Steigrohr ausgebildete Steigleitung 18 erstreckt sich vom Bodenbereich des Behälters 2 innerhalb des Kragens 15 durch den Boden 12 des Aufsatzes 3 bis zur Verdampfungsschale 8, wobei es mit seinem oberen Ende etwas über den Boden der Verdampfungsschale 8 vorsteht. Der Aufsatz 3 dichtet damit die Öffnung 13 des Behälters 2, abgesehen von dem Steigrohr 18 und der Gasaustrittsöffnung 16, hermetisch ab.

Durch den Druck des Gases im Gasraum 17 strömt die Duftstofflösung 5 aus dem Behälter 2 über das Steigrohr 18 in die Verdampfungsschale 8. Die Geschwindigkeit, mit der die Duftstofflösung über das Steigrohr 18 aus dem Behälter 2 verdrängt wird, hängt von der Stromstärke ab, mit der die elektrochemische Gasentwicklungszelle 3 beaufschlagt wird.

Zum Transport der Kartusche 1 im Liegen kann das aus der Verdampfungsschale 8 vorstehende Ende des Steigrohres 18 mit einem nicht dargestellten Stöpsel oder dergleichen verschlossen sein.

In der Umfangswand 9 des Aufsatzes 3 ist ein Schlitz 21 vorgesehen, der zu einem Freiraum 22 unterhalb der Verdampfungsschale 8 führt. Über den Schlitz 21 ist eine Zunge 23.1 in den Freiraum 22 einführbar (Figur 2). Die Zunge 23.1 ist an ihrer Oberseite mit einer als Platine ausgebildeten elektrischen Widerstandsheizung 24 versehen. Ferner weist sie einen Kontakt 25 zur Stromversorgung der elektrochemischen Gasentwicklungszelle 7 auf.

Die Verdampfungsschale 8 weist ferner einen Überlauf 26 auf, der mit einem Auffangbehälter 27 in dem Aufsatz 3 verbunden ist, um zu verhindern, dass die relativ aggressive Duftstofflösung über den Schlitz 21 in den Aufsatz 3 eindringen kann, wenn die Verdampfungsgeschwindigkeit der Duftstofflösung geringer als deren Zufluss über das Steigrohr 18 in die Verdampfungsschale 8 ist.

Wie in Figur 3 dargestellt, sind in einem Außengehäuse 28 an der Innenwand Zungen 23.1 und 23.2 befestigt, um an die Beduftungsvorrichtung zwei in Figur 3 gestrichelt dargestellte Kartuschen 1 anschließen zu können. Die eine Kartusche 1 kann dabei als Reserve dienen, wenn die andere leer ist. Auch können beide Kartuschen 1 unterschiedliche Duftstoffe enthalten. Schließlich kann die Duftstoffabgabe verdoppelt werden, wenn beide Kartuschen gleichzeitig in Betrieb genommen werden.

Oberhalb der Kartusche 1 ist in dem Gehäuse 28 ein nicht dargestellter Ventilator 37 angeordnet, der den Duftstoff aus dem Gehäuse 28 bläst.

Unterhalb der Kartuschen 1 sind in dem Außengehäuse 28 an einer Platte 29 mehrere Drehknöpfe, Schalter und dergleichen 31 sowie ein Display 35 vorgesehen. Mit den Drehknöpfen und Schaltern 31 wird die Vorrichtung ein- und ausgeschaltet. Ferner kann damit über eine nicht dargestellte Steuerschaltung die Stromstärke eingestellt werden, mit der die Heizeinrichtung 24 und die elektrochemische Gasentwicklungszelle 7 über den Kontakt 25 sowie der Ventilator 37 beaufschlagt werden. Mit einem Kabel 34 ist die Vorrichtung an das Stromnetz anschließbar. Das Außengehäuse 28 ist als Säule auf einem plattenförmigen Standfuß 36 angeordnet. Die in dem Gehäuse 28 zum Auswechseln der Kartuschen 1 vorgesehene Öffnung 38 ist durch einen nicht dargestellten Verschlussdeckel verschließbar.

Die Kartusche 1 weist gemäß Figur 1 und 2 einen Griff 39 auf, um sie beim Aufstecken auf die Zungen 23.1, 23.2 bzw. beim Abziehen von derselben besser halten zu können.

## Patentansprüche

1. Vorrichtung zur Abgabe von Duftstoffen mit einem Gehäuse (28) mit einem Vorratsbehälter (2) für die Duftstofflösung (5), einer mit einer elektrischen Heizeinrichtung (24) erwärmbaren Verdampfungsschale (8), und einer elektrochemischen Gasentwicklungszelle (7), durch die die Duftstofflösung (5) durch Verdrängung aus dem Vorratsbehälter (2) der Verdampfungsschale (8) zugeführt wird, **dadurch gekennzeichnet, dass** der Vorratsbehälter (2) mit der Duftstofflösung (5), die elektrochemische Gasentwicklungszelle (7) und die Verdampfungsschale (8) zusammen als austauschbare Kartusche (1) ausgebildet sind und das Gehäuse (28) wenigstens eine solche austauschbare Kartusche (1) aufnimmt.

2. Vorrichtung nach Anspruch 1, **dadurch gekennzeichnet, dass** die Kartusche (1) aus dem Vorratsbehälter (2) und einem damit verbundenen Aufsatz (3) besteht, wobei der Aufsatz (3) die elektrochemische Gasentwicklungszelle (7) enthält und an seiner Oberseite die Verdampfungsschale (8) aufweist.

3. Vorrichtung nach Anspruch 2, **dadurch gekennzeichnet, dass** der Vorratsbehälter (2) an seiner Oberseite (11) eine Öffnung (13) aufweist, über die die elektrochemische Gasentwicklungszelle (7) in dem Aufsatz (3) zur Verdrängung der Duftstofflösung (5) mit dem Vorratsbehälter (2) verbunden ist.

4. Vorrichtung nach Anspruch 3, **dadurch gekennzeichnet, dass** durch die Öffnung (13) in dem Vorratsbehälter (2) eine Steigleitung (18) aus dem Vorratsbehälter (2) durch den Aufsatz (3) zu der Verdampfungsschale (8) führt.

5. Vorrichtung nach einem der Ansprüche 2 bis 4, **dadurch gekennzeichnet, dass** der Aufsatz (3) die Öffnung (13) des Vorratsbehälters (2), abgesehen von der Steigleitung (18), gasdicht abdichtet.

6. Vorrichtung nach einem der Ansprüche 2 bis 5, **dadurch gekennzeichnet, dass** die Verdampfungsschale (8) einen mit einem Auffangbehälter (27) im Aufsatz (3) verbundenen Überlauf (26) aufweist.

7. Vorrichtung nach einem der vorstehenden Ansprüche, **dadurch gekennzeichnet, dass** das Gehäuse (28) die Energieversorgung und die Steuerschaltung aufnimmt.

8. Vorrichtung nach Anspruch 2, **dadurch gekennzeichnet, dass** der Aufsatz (3) unterhalb der Verdampfungsschale (8) einen Freiraum (22) aufweist, in den die elektrische Heizeinrichtung (24) zur Erwärmung der Verdampfungsschale (8) einführbar ist.

9. Vorrichtung nach Anspruch 8, **dadurch gekennzeichnet, dass** die elektrische Heizeinrichtung (24) auf einer in den Freiraum (22) einführbaren Zunge (23.1, 23.2) angeordnet ist.

10. Vorrichtung nach einem der Ansprüche 7 bis 9, **dadurch gekennzeichnet, dass** die elektrochemische Gasentwicklungszelle (7) und/oder die elektrische Heizeinrichtung (24) an die Energieversorgung in dem Gehäuse (28) angeschlossen ist.

11. Vorrichtung nach Anspruch 10, **dadurch gekennzeichnet, dass** die elektrochemische Gasentwicklungszelle (7) über die Zunge (23.1, 23.2)) an die Energieversorgung anschließbar ist.

12. Vorrichtung nach einem der Ansprüche 9 bis 11, **dadurch gekennzeichnet, dass** die Zunge (23.1, 23.2) an dem Gehäuse (28) befestigt ist.

13. Vorrichtung nach Anspruch 1 und 12, **dadurch gekennzeichnet, dass** für jede austauschbare Kartusche (1) in dem Gehäuse (28) eine Zunge (23.1, 23.2) vorgesehen ist.

14. Vorrichtung nach Anspruch 1, **dadurch gekennzeichnet, dass** über der Verdampfungsschale (8) ein Ventilator (37) an dem Gehäuse (28) befestigt ist.
